# EUROPEAN PATENT SPECIFICATION

(11) **EP 3 688 769 B1**
(45) Date of publication and mention of the grant of the patent: **08.11.2023**
(21) Application number: 18779341.9
(22) Date of filing: 25.09.2018
(51) Int. Cl.: G16H 40/63, G16H 40/67, G16H 30/20

(54) **AUTOMATED ASSISTANCE TO STAFF AND QUALITY ASSURANCE BASED ON REAL-TIME WORKFLOW ANALYSIS**
AUTOMATISIERTE UNTERSTÜTZUNG DER MITARBEITER UND QUALITÄTSSICHERUNG AUF BASIS VON ECHTZEIT-WORKFLOW-ANALYSEN
ASSISTANCE AUTOMATISÉE AU PERSONNEL ET ASSURANCE QUALITÉ BASÉE SUR L'ANALYSE EN TEMPS RÉEL DU FLUX DE TRAVAIL

(30) Priority: 26.09.2017 US 201762563127 P
(43) Date of publication of application: 05.08.2020
(73) Proprietor: Koninklijke Philips N.V., 5656 AG Eindhoven (NL)
(72) Inventor: AMTHOR, Thomas, Erik, 5656 AE Eindhoven (NL); NORDHOFF, Tanja, 5656 AE Eindhoven (NL); SCHMIDT, Joachim, 5656 AE Eindhoven (NL); BORGERT, Jörn, 5656 AE Eindhoven (NL); GRAESSLIN, Ingmar, 5656 AE Eindhoven (NL)
(74) Representative: Philips Intellectual Property & Standards
(86) International application number: PCT/EP2018/076018
(87) International publication number: WO 2019/063567

(56) References cited:
- WO-A1-2017/080892
- US-A1- 2014 364 720
- Anonymous: "Finite-state machine - Wikipedia", , 14 September 2017 (2017-09-14), XP055529332, Retrieved from the Internet: URL:https://en.wikipedia.org/w/index.php?t itle=Finite-state_machine&oldid=800585562 [retrieved on 2018-12-03]

## Description

### FIELD

The following relates to the medical arts, clinical decision support arts, automated medical data analytics arts, and related arts.

### BACKGROUND

A difficulty with expansion of the use of complex medical imaging systems is the need for trained operators. Currently, developing countries (e.g., Brazil) may not have access to complex imaging systems, such as Magnetic Resonance (MR), computed tomography (CT), positron emission tomography (PET), single photon emission computed tomography (SPECT), ultrasound (US), and the like. Even if such countries have these systems, medical personnel in these countries may not have proper training or skills to operate these devices. While the problem is less severe in developed countries, wait times for a medical imaging session can still be lengthy, and this can be especially problematic in time-critical medical situations such as a rapidly spreading cancer or a person suffering from a broken (or suspected broken) bone. In many medical imaging laboratories, limited personnel availability results in medical imaging devices being used only during normal ("day shift") working hours, introducing an inherent inefficiency as valuable medical imaging devices are not utilized during off-hours (e.g., during the "night shift").

In the case of developing countries, remote command centers may be set up in these countries to oversee or supervise and train medical personnel to use these devices. However, these command centers can be expensive, time-consuming, and may not be staffed well.

Another difficulty with existing medical imaging procedures relates to documentation of executed imaging procedures. These imaging procedures should be documented for use in future operations, and/or to establish an auditable record of imaging procedures, for training purposes, and/or so forth. Currently, machine logs are maintained in many cases, but these logs are generally at a low level and may not be easily related to specific imaging sessions.

WO2017/080892 discloses a MRI system which can provide guidance to an operator conducting an examination (see claim 1). US2014/364720 also discloses an imaging system providing guidance to an operator for setting up and/or performing subsequent scans.

The following discloses new and improved systems and methods to overcome these problems.

### SUMMARY

The invention is defined by the features of independent claims 1 and 10, while dependent claims contain further advantageous embodiments.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
FIGURE 1 diagrammatically shows an imaging system according to one aspect; and
FIGURE 2 shows an exemplary flow chart operation of the imaging system of FIGURE 1.

### DETAILED DESCRIPTION

The following relates to a system for providing guidance to the medical imaging device operator during a medical imaging examination and creating a quality assurance (QA) record of the medical imaging examination in furtherance of operator training, data mining to improve workflow, post-examination review, and/or so forth.

The illustrative system represents a magnetic resonance imaging (MRI) examination workflow as a state machine. This flexible representation allows for representing the extensive run-to-run variability by providing states and state transitions to represent various pathways that may be followed in any MRI examination instance. For example, states and state transitions can be created to represent events such as patient motion during a scan (e.g., a transition that returns to the same state to repeat the scan), different orders of performing scans, and so forth. Each state is defined by a certain space of values for the state variables (which may store patient parameters, imaging device parameters, examination progress information, or so forth), and each state may have a range of actions that are performed when that state is reached (e.g., displaying instructional video, providing recommendations to the operator, or so forth).

To capture the quality assurance recordation aspect, each state also may have feedback fields that are filled in when the examination enters the state. These may be filled in by way of user input received via user graphical user interface (GUI) dialogs, or by reading automatically generated machine log entries, or by reading patient vital sign sensors, and/or so forth. In one contemplated aspect, the state machine is designed to implement the expected work flow, and if a particular MRI examination deviates from that flow then the operator may be asked to provide feedback as to the reason for the deviation. Such feedback may be solicited, for example, if the operator does not follow a recommendation provided by the system, or if a task takes longer than expected, or if an unusual event occurs, and/or so forth.

The system may be implemented on the scanner host computer and/or on a remote application server. A GUI may be provided via which an expert MRI technologist drafts the state diagram of the state machine including defining the states and various state transitions. Thereafter, in a machine learning phase this state machine is applied to historical MRI examination data. The machine learning provides for determining probabilities of the various state transitions (thus capturing probabilities of various events occurring as events correspond to state transitions), and also provides an efficient mechanism for detecting situations that are not represented by the current state diagram (the expert MRI technologist can then update the state diagram to account for these situations).

After creating the state machine, it is used to provide operator assistance and QA recordation during MRI examinations. The operator experiences the system by way of receiving recommendations, e.g. for customizing the exam card, performing various operations during the examination, or so forth) and being asked to provide feedback at certain points. In execution, the system receives input data about the patient from the Health Information System (HIS) or other medical database(s), and input data about the medical imaging device state by reading the automatically generated machine logs, and input data about the intended examination (e.g. the clinical question being answered) from the MRI exam order in the Radiology Information System (RIS), and/or so forth.

MRI is described as an illustrative medical imaging device to which the disclosed improvements may be usefully applied. However, the disclosed systems and methods are not limited to MRI but rather extends to medical imaging devices generally, e.g. MRI, CT, PET, SPECT, et cetera.

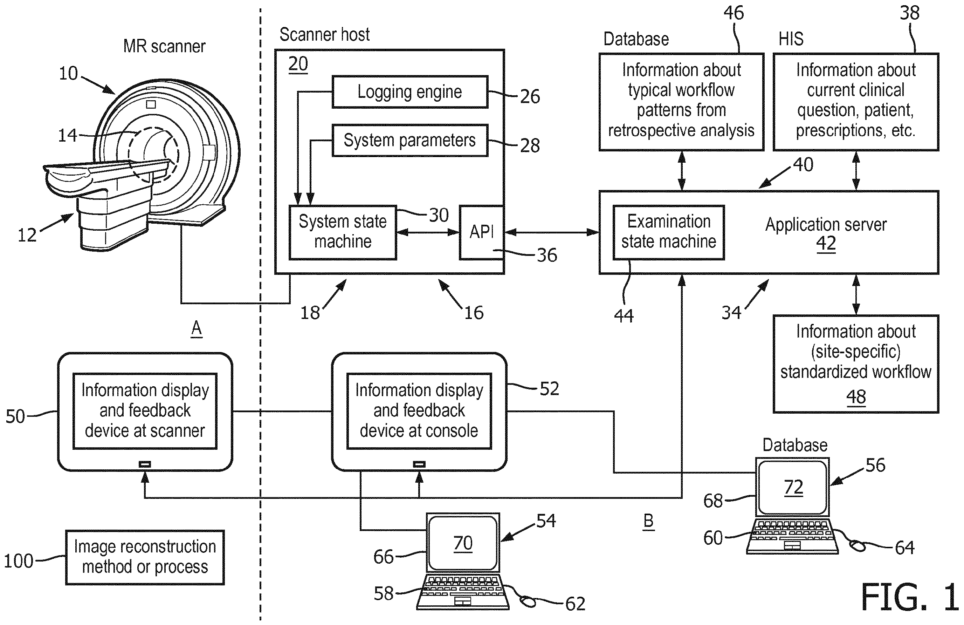

With reference to FIGURE 1, an illustrative medical imaging system **10** is shown. As shown in FIGURE 1, the system **10** includes an image acquisition device **12.** In one example, the image acquisition device **12** can comprise a Magnetic Resonance (MR) imaging device arranged to collect imaging data from a patient disposed in an examination region **14.** However, it will be appreciated that the image acquisition device **12** can include any suitable imaging device (e.g., a computed tomography (CT) device, a positron emission tomography (PET) device, a gamma camera for a single photon emission computed tomography (SPECT) device, and an ultrasound (US) device). The image acquisition device **12** is configured to obtain images of a patient during an image acquisition procedure **100** using known MRI, CT, PET, SPECT, US, et cetera imaging data acquisition and image reconstruction methodologies.

The imaging system **10** also includes a scanner host or device controller **16** operatively connected with the MR scanner **12.** The scanner host **16** can be implemented as a computer or workstation or other electronic data processing device **18** with typical components, such as at least one electronic processor **20.** The device controller **16** also includes a machine logging engine or machine log **26** which stores an operating history (e.g., number of scans, patients scanned, accessory installation/removal events, and the like) of the MR scanner **12.** While a single machine log **26** is shown, it will be appreciated that the machine logs may be organized into two or more logs, e.g. a machine log storing low level machine events, a service log storing servicing events, a laboratory log storing higher level information on patient imaging sessions, et cetera. The device controller **16** also includes a system parameters database **28** storing parameters of the MR scanner **12** (e.g., operating times, coil status, and the like). The at least one electronic processor **20** is programmed to operate the image acquisition device **12** to acquire medical images of a patient, and to maintain a machine log storing an operating history of the image acquisition device in the machine log **26.**

The at least one electronic processor **20** is further programmed to implement a state machine **30** having a plurality of states defined by values of state variables. The states represent respective attainable states of the image acquisition device **12.** The state machine **30** is configured to transition between states during operation of the MR scanner **12.** The state machine **30** represents a current state of the image acquisition device **12.** The values of the state variables of the state machine **30** are determined based at least on content of the machine log **26** for the image acquisition device **12.** The state variables of the state machine **30** can include a patient position, settings of the MR scanner **12** retrieved from the system parameters database **28,** patient vital signs retrieved from sensors (not shown) optionally attached to the patient for certain imaging procedures, patient motion during an imaging scan detected by such sensors or by other approaches such as detecting changes in images (e.g. image frames) acquired from one time interval to the next, different orders of performing scans, and so forth.

The imaging system **10** also includes a server computer **34** in communication with the device controller **16** to receive information from the device controller via an application programming interface (API) **36** disposed on the device controller. The server computer **34** is programmed to retrieve patient information from at least one health information system (HIS) **38** (e.g., an electronic health record (EHR), an electronic medical record (EMR), a cardiovascular information system (CVIS), a picture archiving and communication system (PACS), a radiology information system (RIS), and the like). The server computer **34** can be implemented as a computer or workstation or other electronic data processing device **40** with typical components, such as at least one electronic processor **42.**

The server computer **34** is programmed to model a current state of the image acquisition procedure **100** based at least on content of the patient information retrieved by the server computer from the at least one HIS and data stored in the machine log **26.** To do so, the at least one electronic processor **42** is programmed to implement an examination state machine **44** having a plurality of states by values of state variables. The states represent respective attainable states of the image acquisition procedure **100.** The state machine **44** is configured to transition between states during the image acquisition procedure **100.** The state machine **44** represents a current state of the image acquisition procedure **100.** The values of the state variables of the state machine **44** are determined based at least on patient information retrieved by the server computer **34** from the at least one HIS **38** and/or the state of the state machine **30** of the device controller **16.** The state variables of the state machine **44** include at least an identification of the image acquisition procedure **100** and patient medical information retrieved from the at least one HIS **38.** In some examples, the retrieved patient medical information can include at least a state of patient motion during an imaging scan, a state of different orders of performing imaging scans, patient vital signs, a type of ongoing examination, an exact state of an ongoing image acquisition workflow, a probability for different actions to be performed next, scans that have been performed, scans that still need to be performed, actions still necessary to be performed, descriptions manuals, and guidelines for future steps in the image acquisition procedure **100,** operations to follow and document to achieve quality assurance, and so forth.

In some embodiments, the server computer **34** is also in communication with one or more databases, including a first database **46** storing information about typical imaging workflow patterns from previous image acquisitions procedures, and a second database **48** including information about imaging site-specific standardized workflows information about the patient being scanned during the image acquisition procedure **100.** The state machine **44** is updated from information from the first database **46** and/or the second database **48.**

It will be appreciated that, in some embodiments, a first state machine (i.e., the state machine **30)** and a second state machine (e.g., the state machine **44)** can be implemented on either the device controller **16** or the server computer **34.** The illustrative implementation as two separate state machines **30, 44,** one of which is a system state machine **30** representing the state of time imaging device **12** and the other of which is an examination state machine **44** representing the state of the imaging examination, has certain advantages. For example, this division allows the system state machine **30** to be implemented on the scanner host **16** using information mostly or entirely local to the imaging laboratory, while the examination state machine **44** is implemented at the server computer **34** which has high bandwidth access to the various databases **38, 46, 48** and can leverage the typically greater computing capacity of the server computer **34** (which may, in some embodiments, comprise a network of servers, an ad hoc cloud computing resource, or so forth). The division into separate state machines **30, 44** also facilitates maintaining operational isolation of the scanner host **16** from the hospital-level computing network, which promotes cyber security for the scanner host **16.**

The imaging system **10** also includes at least one feedback device. As shown in FIGURE 1, the at least one feedback device includes a first feedback device **50** and a second feedback device **52.** One or both of the feedback devices **50** and **52** can comprise a computer or workstation or other electronic data processing device **54, 56** with typical components, include at least one corresponding electronic processor **58, 60,** at least one user input device (e.g., a mouse, a keyboard, a trackball, and/or the like) **62, 64,** and a display device **66, 68** (such as an LCD display, projection display, or so forth). In some embodiments, the display devices **66, 68** can be separate components from the corresponding feedback devices **50, 52.** In other embodiments, the display devices **66, 68** can be touch screens, and thus the user input devices **62, 64** constitute a finger movement (e.g., tapping or swiping) by the operator. Each of the display devices **66, 68** is configured to display a corresponding graphical user interface (GUI) **70, 72** including at least one GUI dialog (not shown) to receive inputs (e.g., from the user input devices **62, 64**) from the operator.

The feedback devices **50** and **52** are configured to provide guidance to an operator for performing the image acquisition procedure **100** based on a current state of at least one of the state machines **30** and **44.** For example, the states of the state machine **30** and/or the state machine **44** (or a single state machine) may be extant if one of the state variables is within a certain space. The states machines **30** and **44** each have associated actions to perform when one of the corresponding states is extant. The associated actions can include recommended actions, including one or more of displaying an instructional video to the operator, providing a recommendation (e.g., audio or visual) in the form of a message to the operator. The recommendations can include, for example, images or videos explaining how to position the patient for the specific examination to be performed; images or videos explaining how to administer a contrast agent when one of the state machines **30, 44** identifies an examination requiring the contrast agent and after all scheduled non-contrast agent enhanced scans are finished; information about the required contrast agent dose for the specific patient; advice about special assistance needed for a specific patient; proposals of what to say to the patient in a certain situation (e.g., when one of the state machines **30, 44** detects a repeated scan, so the patient should be calmed down to minimize motion); automatic translation of these proposals to a language spoken by the patient, preferably combined with a speech processing system in case the operator is not speaking the patient's language; proposals for a different scan type that is less sensitive to motion but still allows for the required diagnosis (when one of the state machines **30, 44** detects a repetition of a motion-sensitive scan), and so forth.

In some embodiments, these recommendations can be based on the information stored in one or more of the first and second databases **46, 48.** The GUIs **70, 72** are configured to display the GUI dialogs to receive inputs from the operator indicative of the recommended actions (e.g., "accepted recommendation;" "reject recommendation"; "request recommendation"; "perform scan;" "pause/resume scan" and so forth). In some examples, for each step in the image acquisition procedure **100,** the operator can be asked to acknowledge that the procedure has been performed as proposed by the imaging system **10,** or give a reason for a deviation from this proposal. When the operator does not follow the recommended actions, the GUIs **70, 72** can display a GUI dialog to receive a value via the inputs devices **62, 64** for a feedback field explaining why the recommended action was not followed. This feedback can be stored (e.g., in the one of the databases **46, 48**) to establish a quality control system to document if certain quality related aspects or necessary steps/procedures have been followed by the operator during the examination. This guarantees a standardization of the process steps and increases reproducibility of the workflow, which also leads to an optimization of the workflow. Examples of feedback can include: an acknowledgement by the operator that the patient is positioned in exactly the way shown in an image on the display devices **66, 68** when a certain patient positioning should be used (e.g., for a shoulder or knee examination); an acknowledgement by the operator that the patient is correctly positioned in the image acquisition device **12** (e.g., the patient's left arm is in the imaging region, and not the right arm); a confirmation by the operator that a certain procedure has been explained to the patient; a request to the operator that extra checks are performed on the images according to a global/local quality assurance system; a request to the operator to initiate extra automated image quality checks (e.g. to detect if too much motion etc.), to be able to re-scan the patient immediately, opposed to calling him in at a later point in time, and so forth. When these feedbacks are combined with information from the databases **46, 48,** the imaging system **10** can check or ask the operator to check that each element defined in the standardized workflow have been considered. Where feasible, such manually provided feedback can be cross-referenced with available machine log information and/or sensor data, e.g. if the operator asserts an operation has been performed but machine log entries are not consistent with this assertion, then follow-up feedback questions may be asked, and/or the discrepancy may be stored as part of the QA information.

The imaging system **10** can be configured in other ways. In one embodiment, to minimize an integral cost of the implementation (e.g., lower hardware cost, easier maintenance cost by using use of service access to the MR system, etc.) of the imaging system **10,** the real-time analysis of the feedback devices **50, 52** is executed on the device controller **16,** by retrieving additional required information via the API **36** to other components such as the databases **46, 48** databases and/or the HIS **38.** The displays **66, 68** of the feedback devices **50, 52** are connected to the device controller **16** or by the server computer **34.** In another example, the first feedback device **50** is built directly into the image acquisition device **12.**

In another embodiment, the server computer **34** is also connected to a remote command center (not shown). Information about the state of the examination can thus be transferred automatically to a remote consultant. When a communication channel to the remote command center is opened on request of the operator, the necessary information about the examination state is already available. In another example, the feedback devices **50, 52** serve as communication devices with a remote consultant (not shown), e.g. by providing an audio or video connection. In another embodiment, the quality-control-related information from the feedback devices **50, 52** is transferred into a hospital IT system (not shown) to allow for hospital-wide access, documentation, and archiving either by means of unsolicited messages or on request (query).

In a more specific embodiment, as further shown in FIGURE 1, the imaging system **10** includes the image acquisition device **12,** the device controller **16,** the server computer **34,** the databases **46** and **48** and the first and second feedback devices **50** and **52.** In some examples (e.g., when the image acquisition device **12** is an MR scanner), the MR scanner, and the first feedback device **50** can be disposed in a first room **A** with a radio-frequency (RF) shield (indicated by the dashed line) in the walls of the first room. The device controller **16,** server computer **34,** the databases **46, 48,** and the second feedback device **52** can be disposed in a separate second room **B** that does not include a RF shield. In another example, the device controller **16** and the server computer **34** may be disposed in separate, non-RF shielded rooms. Advantageously, the server computer **34,** the databases **46, 48,** and the second feedback device **52** are protected from RFs emitted by the MR scanner **12.** In one embodiment, information about deviations from the standard workflow is used for adaptively changing and re-optimizing the room plan.

The at least one electronic processor **20** of the device controller **16** and/or the electronic processor **42** of the server computer **34** is/are operatively connected with a non-transitory storage medium (not shown) that stores instructions which are readable and executable by the at least one electronic processor(s) **20** (and/or **42**) to perform disclosed operations including performing the image acquisition method or process **100.** The non-transitory storage medium may, for example, comprise a hard disk drive, RAID, or other magnetic storage medium; a solid state drive, flash drive, electronically erasable read-only memory (EEROM) or other electronic memory; an optical disk or other optical storage; various combinations thereof; or so forth. In another embodiment, the image acquisition method or process **100** may be performed by cloud processing.

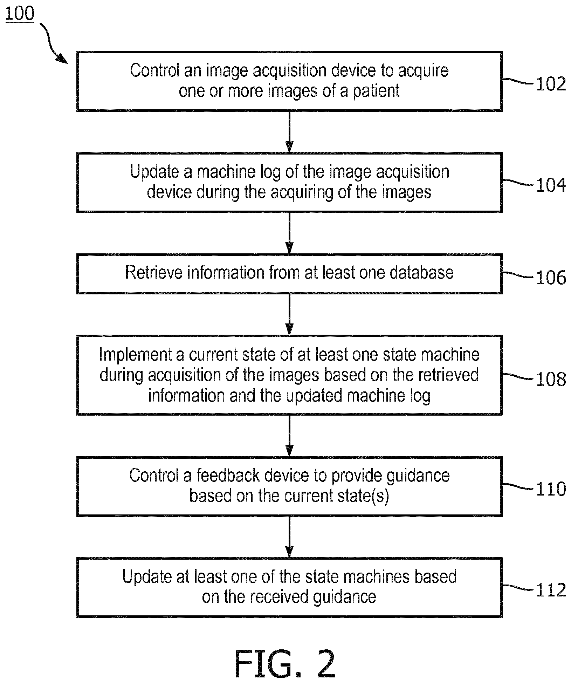

With reference to FIGURE 2, an illustrative embodiment of the image acquisition method **100** is diagrammatically shown as a flowchart. At **102,** the at least one electronic processor **20, 44** is programmed to control the image acquisition device **12** to acquire one or more images of a patient. At **104,** the at least one electronic processor **20, 44** is programmed to update the machine log **26** during the acquiring of the images. At **106,** the at least one electronic processor **20, 44** is programmed to retrieve information from at least one of the HIS **38,** the first database **46,** and the second database **48.** At **108,** the at least one electronic processor **20, 44** is programmed to implement a current state of at least one of the state machines **30, 44** during acquisition of the images based on the retrieved information and the updated machine log **26.** At **110,** the at least one electronic processor **20, 44** is programmed to control at least one of the feedback devices **50, 52** to provide guidance to an operator of the image acquisition device **12** based on the current state of at least one of the state machines **30, 44.** At **112** the at least one electronic processor **20, 44** is programmed to update at least one of the state machines **30, 44** as the state machine(s) transition between states based on the received guidance.

The disclosure has been described with reference to the preferred embodiments. Modifications and alterations may occur to others upon reading and understanding the preceding detailed description. It is intended that the invention be construed as including all such modifications and alterations insofar as they come within the scope of the appended claims

## Claims

1. An imaging system (**10**), comprising:
an image acquisition device (**12**);
a device controller (**16**) comprising an electronic processor (**20**) programmed to operate the image acquisition device to acquire medical images of a patient and to maintain a machine log (**26**) storing an operating history of the image acquisition device;
a server computer (**34**) programmed to retrieve patient information from at least one health information system, HIS (**18**); and
at least one feedback device (**50, 52**);
wherein the device controller, the server computer, or a combination of the device controller and server computer is programmed to implement at least one state machine (**30**, **44**) having a plurality of states defined by values of state variables wherein the states represent respective attainable states of an image acquisition procedure (**100**) and the image acquisition device, the at least one state machine configured to transition between states during the image acquisition procedure whereby the at least one state machine represents a current state of the image acquisition procedure and the image acquisition device, wherein the values of the state variables of the at least one state machine are determined based at least on content of the machine log for the image acquisition device and patient information retrieved by the server computer from the at least one HIS; and
wherein the at least one feedback device is configured to provide guidance to an operator for performing the image acquisition procedure based on a current state of the at least one state machine, wherein the at least one state machine (**30, 44**) comprises:
a first state machine (**30**) being a system state machine representing the imaging acquisition device (**12**) and having a plurality of states, wherein the values of the state variables of the first state machine are determined based on content of the machine log for the image acquisition device; and
a second state machine (**44**) being an examination state machine representing the image acquisition procedure (**100**) and having a plurality of states, wherein the values of the state variables of the second state machine are determined based at least on the first state machine and patient information retrieved by the server from the at least one HIS (**18**).

2. The imaging system (**10**) of claim 1, wherein:
the state variables of the first state machine (**30**) include at least a patient position and settings of the image acquisition device (**12**), and
the state variables of the second state machine (**44**) include at least an identification of the image acquisition procedure and patient medical information retrieved from the at least one HIS (**18**).

3. The imaging system (**10**) of claim 1, wherein the at least one state machine (**30**, **44**) has a plurality of states including at least a state of patient motion during an imaging scan and a state of different orders of performing imaging scans.

4. The imaging system (**10**) of claim 1, wherein the states of the at least one state machine (**30, 44**) have associated actions to be performed when one of the corresponding states is extant, wherein the associated actions include at least displaying an instructional video to the operator and providing recommendations to the operator.

5. The imaging system (**10**) of claim 4, wherein the associated actions include recommended actions and, conditional upon the operator not following a recommended action,
wherein the at least one feedback device (**50, 52**) is configured to display a graphical user interface, GUI, (**70, 72)** including at least one GUI dialog configured to receive a value for a feedback field explaining why the recommended action was not followed.

6. The imaging system (**10**) of claim 3, wherein the state variables further include patient vital sign values.

7. The imaging system (**10**) of any one of claims 4-6, wherein the at least one feedback device (**50, 52**) is configured to display a graphical user interface, GUI, (**70, 72**) including at least one GUI dialog configured to receive an input by the operator indicative of at least one of the recommended actions.

8. The imaging system (**10**) of claim 1, further including at least one database (**46**, **48**) operatively connected with the server computer (**34**), the at least one database storing information related to image acquisition workflow patterns from previous image acquisition procedures, information about standardized workflows of the image acquisition device (**12**), and information about the patient being scanned during the image acquisition procedure,
wherein the second state machine (**44**) is updated with information from the at least one database, wherein the at least one feedback device (**50, 52**) is configured to provide recommendations to the operator during the image acquisition procedure by at least one of audio or visual message, the recommendations being based on the information in the at least one database (**46, 48**).

9. The imaging system (**10**) of any one of claims 1-8, wherein the image acquisition device (**12**) is one of a magnetic resonance imaging, MRI, (MRI) device, a computed tomography, CT, device, a positron emission tomography, PET, device, a single photon emission computed tomography, SPECT, device, or an ultrasound, US, device;
wherein the at least one feedback device (**50, 52**) includes a first feedback device (**50**) and a second feedback device (**52**), and
wherein the first feedback device, and the image acquisition device are located in a first, radio-frequency,RF, shielded room (**A**), and the device controller (**16**), the second feedback device and the image acquisition application server (**34**) are located in a second separate, non-RF shielded room (**B**).

10. A non-transitory computer-readable medium storing instructions readable and executable by a computer (**16, 34**) including at least one electronic processor (**20, 44**) to perform an image acquisition method (**100**), the method comprising:
controlling an image acquisition device (**12**) to acquire one or more images of a patient;
updating a machine log (**26**) of the image acquisition device during the acquiring of the images;
retrieving information from at least one of a health information system, HIS, (**38**)**,** a first database (**46**) storing information about typical imaging workflow patterns from previous image acquisitions procedures, and a second database (**48**) including information about imaging site-specific standardized workflows information about the patient being scanned;
implementing a current state of at least one state machine **(30, 44)** of the computer during acquisition of the images based on the retrieved information and the updated machine log;
controlling at least one feedback device **(50, 52)** operatively connected with the image acquisition device and the computer to provide guidance to an operator of the image acquisition device based on the current state of at least one of the state machines; and
updating at least one of the state machines as the at least one state machine transitions between states based on the received guidance, wherein the at least one state machine (**30**, **44**) comprises:
a first state machine (**30**) being a system state machine representing the imaging acquisition device (**12**) and having a plurality of states, wherein the values of the state variables of the first state machine are determined based on content of the machine log for the image acquisition device; and
a second state machine (**44**) being a examination state machine representing the image acquisition procedure (**100**) and having a plurality of states, wherein the values of the state variables of the second state machine are determined based at least on the first state machine and patient information retrieved by the server from the at least one HIS (**38**).

11. The non-transitory computer-readable medium of claim 10, wherein:
the state variables of the first state machine (**30**) include at least a patient position and settings of the image acquisition device (**12**), and
the state variables of the second state machine (**44**) include at least an identification of the image acquisition procedure and patient medical information retrieved from the at least one HIS (**18**).

12. The non-transitory computer-readable medium of claim 10, wherein the at least one state machine (**30, 44**) has a plurality of states including at least a state of patient motion during an imaging scan and a state of different orders of performing imaging scans; and
wherein the states of the at least one state machine have associated actions to be performed when one of the corresponding states is extant, the associated actions include at least displaying an instructional video to the operator, providing recommendations to the operator, and recommended actions.

13. The non-transitory computer-readable medium of claim 10, further including at least one database (**46**, **48**) operatively connected with the computer (**16, 34**), the at least one database storing information related to image acquisition workflow patterns from previous image acquisition procedures, information about standardized workflows of the image acquisition device (**12**), and information about the patient being scanned during the image acquisition procedure,
wherein the second state machine (**44**) is updated with information from the at least one database; and
wherein the at least one feedback device (**50, 52**) is configured to provide recommendations to the operator during the image acquisition procedure by at least one of audio or visual message, the recommendations being based on the information in the at least one database.

## Patentansprüche

1. Bildgebungssystem (**10**), das Folgendes umfasst:
eine Bildaufnahmevorrichtung (**12**);
eine Vorrichtungssteuervorrichtung (**16**), die einen elektronischen Prozessor (**20**) umfasst, der dazu programmiert ist, die Bildaufnahmevorrichtung zu betreiben, um medizinische Bilder eines Patienten aufzunehmen und ein Maschinenprotokoll (**26**) zu führen, dass eine Betriebshistorie der Bildaufnahmevorrichtung speichert; einen Server-Computer (**34**), der dazu programmiert ist, Patienteninformationen von mindestens einem Gesundheitsinformationssystem, HIS (**18**), abzurufen; und mindestens eine Feedback-Vorrichtung (**50, 52**);
wobei die Vorrichtungssteuervorrichtung, der Server-Computer oder eine Kombination aus der Vorrichtungssteuervorrichtung und dem Server-Computer dazu programmiert ist, mindestens eine Zustandsmaschine (**30, 44**) zu implementieren, die eine Vielzahl von Zuständen aufweist, die von Werten von Zustandsvariablen definiert sind, wobei die Zustände jeweilige erreichbare Zustände einer Bildaufnahmevorgehensweise (**100**) und der Bildaufnahmevorrichtung darstellen, die mindestens eine Zustandsmaschine dazu konfiguriert ist, zwischen Zuständen während der Bildaufnahmevorgehensweise überzugehen, wobei die mindestens eine Zustandsmaschine einen aktuellen Zustand der Bildaufnahmevorgehensweise und der Bildaufnahmevorrichtung darstellt, wobei die Werte der Zustandsvariablen der mindestens einen Zustandsmaschine mindestens basierend auf Inhalt des Maschinenprotokolls für die Bildaufnahmevorrichtung und Patienteninformationen, die von dem Server-Computer aus dem mindestens einen HIS abgerufen werden, bestimmt werden; und
wobei die mindestens eine Feedback-Vorrichtung dazu konfiguriert ist, einen Bediener zum Durchführen der Bildaufnahmevorgehensweise basierend auf einem aktuellen Zustand der mindestens einen Zustandsmaschine bereitzustellen, wobei die mindestens eine Zustandsmaschine (**30, 44**) Folgendes umfasst:
eine erste Zustandsmaschine (**30**), die eine Systemzustandsmaschine ist, die die Bildaufnahmevorrichtung (**12**) darstellt und eine Vielzahl von Zuständen aufweist, wobei die Werte der Zustandsvariablen der ersten Zustandsmaschine basierend auf Inhalt des Maschinenprotokolls für die Bildaufnahmevorrichtung bestimmt werden; und eine zweite Zustandsmaschine (**44**), die eine Untersuchungszustandsmaschine ist, die die Bildaufnahmevorgehensweise (**100**) darstellt und eine Vielzahl von Zuständen aufweist, wobei die Werte der Zustandsvariablen der zweiten Zustandsmaschine basierend mindestens auf der ersten Zustandsmaschine und Patienteninformationen, die von dem Server von dem mindestens einen HIS (**18**) abgerufen werden, bestimmt werden.

2. Bildgebungssystem (**10**) nach Anspruch 1, wobei:
die Zustandsvariablen der ersten Zustandsmaschine (**30**) mindestens eine Patientenposition und Einstellungen der Bildaufnahmevorrichtung (**12**) beinhalten, und die Zustandsvariablen der zweiten Zustandsmaschine (**44**) mindestens eine Identifikation der Bildaufnahmevorgehensweise und medizinischer Patienteninformationen beinhalten, die aus dem mindestens einen HIS (**18**) abgerufen werden.

3. Bildgebungssystem (**10**) nach Anspruch 1, wobei die mindestens eine Zustandsmaschine (**30, 44**) eine Vielzahl von Zuständen aufweist, die mindestens einen Zustand der Patientenbewegung während eines Bildgebungsscans und eines Zustands unterschiedlicher Reihenfolgen des Durchführens von Bildgebungsscans beinhaltet.

4. Bildgebungssystem (**10**) nach Anspruch 1, wobei die Zustände der mindestens einen Zustandsmaschine (**30, 44**) assoziierte Aktionen aufweisen, die durchgeführt werden sollen, wenn einer der entsprechenden Zustände existiert, wobei die assoziierten Aktionen mindestens die Anzeige eines Lehrvideos für den Bediener und die Bereitstellung von Empfehlungen für den Bediener beinhalten.

5. Bildgebungssystem (**10**) nach Anspruch 4, wobei die assoziierten Aktionen empfohlene Aktionen abhängig davon, dass der Bediener eine empfohlene Aktion nicht befolgt, beinhaltet,
wobei die mindestens eine Feedback-Vorrichtung (**50, 52**) dazu konfiguriert ist, eine grafische Benutzeroberfläche, GUI, (**70**, **72**) anzuzeigen, die mindestens einen GUI-Dialog beinhaltet, der dazu konfiguriert ist, einen Wert für ein Feedback-Feld zu empfangen, das erklärt, warum die empfohlene Aktion nicht befolgt wurde.

6. Bildgebungssystem (**10**) nach Anspruch 3, wobei die Zustandsvariablen ferner Vitalwerte des Patienten beinhalten.

7. Bildgebungssystem (**10**) nach einem der Ansprüche 4-6, wobei die mindestens eine Feedback-Vorrichtung (**50**, **52**) dazu konfiguriert ist, eine grafische Benutzeroberfläche, GUI, (**70, 72**) anzuzeigen, die mindestens einen GUI-Dialog beinhaltet, der dazu konfiguriert ist, eine Eingabe durch den Bediener, die mindestens eine der empfohlenen Aktionen angibt, zu empfangen.

8. Bildgebungssystem (**10**) nach Anspruch 1, das ferner mindestens eine Datenbank (**46, 48**) beinhaltet, die betriebswirksam mit dem Server-Computer (**34**) verbunden ist, wobei die mindestens eine Datenbank Informationen speichert, die Bildaufnahme-Workflow-Muster aus vorhergehenden Bildaufnahmevorgehensweisen, Informationen über standardisierte Workflows der Bildaufnahmevorrichtung (**12**) und Informationen über den Patienten, der während der Bildaufnahmevorgehensweise gescannt wird, betreffen, wobei die zweite Zustandsmaschine (**44**) mit Informationen aus der mindestens einen Datenbank aktualisiert wird, wobei die mindestens eine Feedback-Vorrichtung (**50, 52**) dazu konfiguriert ist, dem Bediener während der Bildaufnahmevorgehensweise durch mindestens eine akustische oder visuelle Nachricht die Empfehlungen anzugeben, wobei die Empfehlungen auf den Informationen in der mindestens einen Datenbank (**46**, **48**) basieren.

9. Bildgebungssystem (**10**) nach einem der Ansprüche 1-8, wobei die Bildaufnahmevorrichtung (**12**) eine einer Magnetresonanztomographievorrichtung, MRI-Vorrichtung, einer Computertomographievorrichtung, CT-Vorrichtung, einer Positronenemissionstomographievorrichtung, PET-Vorrichtung, einer Einzelphotonenemissions-Computertomographievorrichtung, SPECT-Vorrichtung, oder einer Ultraschallvorrichtung, US-Vorrichtung, ist; wobei die mindestens eine Feedback-Vorrichtung (**50**, **52**) eine erste Feedback-Vorrichtung (**50**) und eine zweite Feedback-Vorrichtung (**52**) beinhaltet, und wobei sich die erste Feedback-Vorrichtung und die Bildaufnahmevorrichtung in einem ersten Hochfrequenz-geschirmten Raum, HF-geschirmten Raum, (**A**), befinden, und sich die Vorrichtungssteuervorrichtung (**16**), die zweite Feedback-Vorrichtung und der Bildaufnahme-Anwendungsserver (**34**) in einem zweiten separaten, nicht Hochfrequenz-geschirmten Raum, nicht-HF-geschirmten Raum (**B**) befinden.

10. Nicht-flüchtiges computerlesbares Medium, das Anweisungen speichert, die von einem Computer (**16, 34**), der mindestens einen elektronischen Prozessor (**20, 44**) beinhaltet, gelesen und ausgeführt werden können, um ein Bildaufnahmeverfahren (**100**) durchzuführen, wobei das Verfahren Folgendes umfasst:
Steuern einer Bildaufnahmevorrichtung (**12**) zum Aufnehmen eines oder mehrerer Bilder eines Patienten; Aktualisieren eines Maschinenprotokolls (**26**) der Bildaufnahmevorrichtung während des Aufnehmens der Bilder;
Abrufen von Informationen aus mindestens einem eines Gesundheitsinformationssystem, HIS, (**38**), einer ersten Datenbank (**46**), die Informationen über typische Bildgebungs-Workflow-Muster aus vorhergehenden Bildaufnahmevorgehensweisen speichert, und eine zweite Datenbank (**48**), die Informationen über bildgebungsortsspezifische standardisierte Workflow-Informationen über den Patienten, der gescannt wird, beinhaltet; Implementieren eines aktuellen Zustands mindestens einer Zustandsmaschine (**30, 44**) des Computers während der Aufnahme der Bilder basierend auf den abgerufenen Informationen und dem aktualisierten Maschinenprotokoll;
Steuern mindestens einer Feedback-Vorrichtung (**50, 52**), die betriebswirksam mit der Bildaufnahmevorrichtung und dem Computer verbunden ist, um einem Bediener der Bildaufnahmevorrichtung basierend auf dem aktuellen Zustand mindestens einer der Zustandsmaschinen Anleitung bereitzustellen; und Aktualisieren mindestens einer der Zustandsmaschinen, während die mindestens eine Zustandsmaschine basierend auf der empfangenen Anleitung zwischen Zuständen übergeht, wobei die mindestens eine Zustandsmaschine (**30, 44**) Folgendes umfasst:
eine erste Zustandsmaschine (**30**), die eine Systemzustandsmaschine ist, die die Bildaufnahmevorrichtung (**12**) darstellt und eine Vielzahl von Zuständen aufweist, wobei die Werte der Zustandsvariablen der ersten Zustandsmaschine basierend auf Inhalt des Maschinenprotokolls für die Bildaufnahmevorrichtung bestimmt werden; und eine zweite Zustandsmaschine (**44**), die eine Untersuchungszustandsmaschine ist, die die Bildaufnahmevorgehensweise (**100**) darstellt und eine Vielzahl von Zuständen aufweist, wobei die Werte der Zustandsvariablen der zweiten Zustandsmaschine mindestens basierend auf der ersten Zustandsmaschine und Patienteninformationen, die von dem Server aus dem mindestens einen HIS (**38**) abgerufen werden, bestimmt werden.

11. Nicht-flüchtiges computerlesbares Medium nach Anspruch 10, wobei: die Zustandsvariablen der ersten Zustandsmaschine (**30**) mindestens eine Patientenposition und Einstellungen der Bildaufnahmevorrichtung (**12**) beinhalten, und die Zustandsvariablen der zweiten Zustandsmaschine (**44**) mindestens eine Identifikation des Bildaufnahmevorgangs und medizinische Patienteninformationen beinhalten, die von dem mindestens einen HIS (**18**) abgerufen werden.

12. Nicht-flüchtiges computerlesbares Medium nach Anspruch 10, wobei die mindestens eine Zustandsmaschine (**30, 44**) eine Vielzahl von Zuständen aufweist, die mindestens einen Zustand der Patientenbewegung während eines Bildgebungsscans und eines Zustands unterschiedlicher Reihenfolgen des Durchführens von Bildgebungsscans beinhaltet; und wobei die Zustände der mindestens einen Zustandsmaschine assoziierte Aktionen aufweisen, die durchgeführt werden sollen, wenn einer der entsprechenden Zustände existiert, wobei die assoziierten Aktionen mindestens das Anzeigen eines Anleitungsvideos für den Bediener, das Empfehlungen für den Bediener bereitstellt, und empfohlene Aktionen beinhalten.

13. Nicht-flüchtiges computerlesbares Medium nach Anspruch 10, das ferner mindestens eine Datenbank (**46**, **48**) beinhaltet, die betriebswirksam mit dem Computer (**16, 34**) verbunden ist, wobei die mindestens eine Datenbank Informationen speichert, die Bildaufnahme-Workflow-Muster aus vorhergehenden Bildaufnahmevorgehensweisen, Informationen über standardisierte Workflows der Bildaufnahmevorrichtung (**12**) und Informationen über den Patienten, der gescannt wird, während der Bildaufnahmevorgehensweise betreffen, wobei die zweite Zustandsmaschine (**44**) mit Informationen aus der mindestens eine Datenbank aktualisiert wird; und wobei die mindestens eine Feedback-Vorrichtung (**50, 52**) dazu konfiguriert ist, dem Bediener während der Bildaufnahmevorgehensweise durch mindestens eine einer akustischen oder visuellen Nachricht Empfehlungen bereitzustellen, wobei die Empfehlungen auf den Informationen in der mindestens eine Datenbank basieren.

## Revendications

1. Système d'imagerie (10) comprenant un dispositif d'acquisition d'images (12);
un contrôleur de dispositif (16) comprenant un processeur électronique (20) programmé pour faire fonctionner le dispositif d'acquisition d'images afin d'acquérir des images médicales d'un patient et pour maintenir un journal de machine (26) stockant un historique de fonctionnement du dispositif d'acquisition d'images;
un ordinateur serveur (34) programmé pour récupérer des informations sur le patient à partir d'au moins un système d'information sur la santé, HIS (18); et
au moins un dispositif de retour d'information (50,52); dans lequel le contrôleur du dispositif, l'ordinateur serveur ou une combinaison du contrôleur du dispositif et de l'ordinateur serveur est programmé pour mettre en œuvre au moins une machine d'état (30,44) ayant une pluralité d'états définis par des valeurs de variables d'état dans lesquels les états représentent des états respectifs réalisables d'une procédure d'acquisition d'image (100) et du dispositif d'acquisition d'image, l'au moins une machine d'état est configurée pour passer d'un état à l'autre au cours de la procédure d'acquisition d'images, l'au moins une machine d'état représentant un état actuel de la procédure d'acquisition d'images et du dispositif d'acquisition d'images, les valeurs des variables d'état de l'au moins une machine d'état étant déterminées en fonction au moins du contenu du journal de la machine pour le dispositif d'acquisition d'images et des informations sur le patient récupérées par l'ordinateur serveur à partir de l'au moins un HIS; et
dans lequel l'au moins un dispositif de rétroaction est configuré pour guider un opérateur dans l'exécution de la procédure d'acquisition d'images sur la base d'un état actuel de l'au moins un automate d'état, dans lequel l'au moins un automate d'état (30,44) comprend:
une première machine d'état (30) étant une machine d'état système représentant le dispositif d'acquisition d'images (12) et ayant une pluralité d'états, dans laquelle les valeurs des variables d'état de la première machine d'état sont déterminées sur la base du contenu du journal de la machine pour le dispositif d'acquisition d'images; et
une deuxième machine d'état (44), qui est une machine d'état d'examen représentant la procédure d'acquisition d'images (100) et comportant plusieurs états, dans laquelle les valeurs des variables d'état de la deuxième machine d'état sont déterminées sur la base au moins de la première machine d'état et des informations sur le patient récupérées par le serveur à partir d'au moins un HIS (18).

2. Système d'imagerie (10) de la revendication 1, dans lequel:
les variables d'état de la première machine d'état (30) comprennent au moins la position du patient et les réglages du dispositif d'acquisition d'images (12), et les variables d'état de la deuxième machine d'état (44) comprennent au moins une identification de la procédure d'acquisition d'images et des informations médicales sur le patient extraites d'au moins un HIS (18) .

3. Système d'imagerie (10) de la revendication 1, dans lequel l'au moins une machine d'état (30,44) possède plusieurs états, dont au moins un état de mouvement du patient pendant un examen d'imagerie et un état de différents ordres d'exécution des examens d'imagerie.

4. Système d'imagerie (10) de la revendication 1, dans lequel les états de l'au moins une machine d'état (30,44) sont associés à des actions à effectuer lorsque l'un des états correspondants est existant, dans lequel les actions associées comprennent au moins l'affichage d'une vidéo d'instruction à l'intention de l'opérateur et la fourniture de recommandations à l'opérateur.

5. Système d'imagerie (10) de la revendication 4, dans lequel les actions associées comprennent des actions recommandées et, sous réserve que l'opérateur ne suive pas une action recommandée, dans lequel au moins un dispositif de retour d'information (50,52) est configuré pour afficher une interface utilisateur graphique GUI,(70,72) comprenant au moins une boîte de dialogue GUI configurée pour recevoir une valeur pour un champ de retour d'information expliquant pourquoi l'action recommandée n'a pas été suivie.

6. Système d'imagerie (10) de la revendication 3, dans lequel les variables d'état comprennent en outre les valeurs des signes vitaux du patient.

7. Système d'imagerie (10) de l'une des revendications 4 à 6, dans lequel l'au moins un dispositif de rétroaction (50, 52) est configuré pour afficher une interface utilisateur graphique, GUI (70,72) comprenant au moins un dialogue GUI configuré pour recevoir une entrée de l'opérateur indiquant au moins l'une des actions recommandées.

8. Système d'imagerie (10) de la revendication 1, comprenant en outre au moins une base de données (46,48) fonctionnellement connectée à l'ordinateur serveur (34), l'au moins une base de données stockant des informations relatives aux modèles de flux de travail d'acquisition d'image des procédures d'acquisition d'image précédentes, des informations sur les flux de travail normalisés du dispositif d'acquisition d'image (12), et des informations sur le patient scanné au cours de la procédure d'acquisition d'image, dans lequel la deuxième machine d'état (44) est mise à jour avec des informations provenant d'au moins une base de données, dans lequel au moins un dispositif de rétroaction (50,52) est configuré pour fournir des recommandations à l'opérateur pendant la procédure d'acquisition d'image par au moins un message audio ou visuel, les recommandations étant basées sur les informations contenues dans au moins une base de données (46,48).

9. Système d'imagerie (10) de l'une des revendications 1 à 8, dans lequel le dispositif d'acquisition d'images (12) est un dispositif d'imagerie par résonance magnétique IRM, un dispositif de tomographie par ordinateur CT, un dispositif de tomographie par émission de positons PET, un dispositif de tomographie par émission d'un seul photon SPECT, ou un dispositif d'échographie US;
dans lequel l'au moins un dispositif de retour (50,52) comprend un premier dispositif de retour (50) et un second dispositif de retour (52), et
dans lequel le premier dispositif de retour et le dispositif d'acquisition d'images sont situés dans une première pièce (A) blindée contre les radiofréquences, RF, et le contrôleur (16), le second dispositif de retour et le serveur d'application d'acquisition d'images (34) sont situés dans une seconde pièce (B) séparée, non blindée contre les RF.

10. Support non transitoire lisible par ordinateur stockant des instructions lisibles et exécutables par un ordinateur (16,34) comprenant au moins un processeur électronique (20,44) pour exécuter une méthode d'acquisition d'image (100), la méthode consistant à:
commander un dispositif d'acquisition d'images (12) pour acquérir une ou plusieurs images d'un patient;
mettre à jour un journal de machine (26) du dispositif d'acquisition d'images pendant l'acquisition des images;
extraire des informations d'au moins un système d'information sur la santé HIS (38), d'une première base de données (46) stockant des informations sur les modèles typiques de flux de travail d'imagerie à partir de procédures d'acquisition d'images antérieures, et une seconde base de données (48) comprenant des informations sur les flux de travail normalisés spécifiques à un site d'imagerie;
mettre en œuvre d'un état actuel d'au moins une machine d'état (30,44) de l'ordinateur pendant l'acquisition des images sur la base des informations récupérées et du journal de la machine mis à jour;
contrôler au moins un dispositif de rétroaction (50,52) fonctionnellement connecté au dispositif d'acquisition d'images et à l'ordinateur pour fournir des conseils à un opérateur du dispositif d'acquisition d'images sur la base de l'état actuel d'au moins une des machines d'état; et
mettre à jour au moins une des machines d'état lorsque la machine d'état au moins passe d'un état à l'autre sur la base des conseils reçus, dans lequel la machine d'état au moins (30,44) comprend:
une première machine d'état (30) étant une machine d'état système représentant le dispositif d'acquisition d'images (12) et ayant une pluralité d'états, dans laquelle les valeurs des variables d'état de la première machine d'état sont déterminées sur la base du contenu du journal de la machine pour le dispositif d'acquisition d'images; et
une deuxième machine d'état (44), qui est une machine d'état d'examen représentant la procédure d'acquisition d'images (100) et comportant plusieurs états, dans laquelle les valeurs des variables d'état de la deuxième machine d'état sont déterminées sur la base au moins de la première machine d'état et des informations sur le patient récupérées par le serveur à partir d'au moins un HIS (38).

11. Le support informatique non transitoire de la revendication 10, dans lequel:
les variables d'état de la première machine d'état (30) comprennent au moins la position du patient et les réglages du dispositif d'acquisition d'images (12), et les variables d'état de la deuxième machine d'état (44) comprennent au moins une identification de la procédure d'acquisition d'images et des informations médicales sur le patient extraites d'au moins un HIS (18) .

12. Le support non transitoire lisible par ordinateur de la revendication 10, dans lequel l'au moins une machine d'état (30,44) a une pluralité d'états comprenant au moins un état de mouvement du patient pendant un balayage d'imagerie et un état de différents ordres d'exécution de balayages d'imagerie; et
dans lequel les états de l'au moins une machine d'état ont des actions associées à effectuer lorsque l'un des états correspondants est existant, les actions associées comprennent au moins l'affichage d'une vidéo d'instruction à l'opérateur, la fourniture de recommandations à l'opérateur, et des actions recommandées.

13. Le support non transitoire lisible par ordinateur de la revendication 10, comprenant en outre au moins une base de données (46,48) connectée de manière opérationnelle à l'ordinateur (16,34), l'au moins une base de données stockant des informations relatives aux modèles de flux d'acquisition d'images des procédures d'acquisition d'images précédentes, des informations sur les flux de travail normalisés du dispositif d'acquisition d'images (12), et des informations sur le patient scanné au cours de la procédure d'acquisition d'images, dans lequel le second automate (44) est mis à jour à l'aide d'informations provenant d'au moins une base de données; et
dans lequel l'au moins un dispositif de rétroaction (50,52) est configuré pour fournir des recommandations à l'opérateur pendant la procédure d'acquisition d'images par au moins un message audio ou visuel, les recommandations étant basées sur les informations contenues dans l'au moins une base de données.
